# EUROPÄISCHE PATENTSCHRIFT

(11) **EP 0 534 096 B1**
(45) Veröffentlichungstag und Bekanntmachung des Hinweises auf die Patenterteilung: **05.11.1997**
(21) Anmeldenummer: 92113169.4
(22) Anmeldetag: 01.08.1992
(51) Int. Cl.: G01N 33/44, G01N 21/90, B08B 9/46

(54) **Verfahren und Vorrichtung zum Untersuchen von Behältnissen auf Fremdstoffe**
Method and device for examining impurities in containers
Procédé et dispositif pour examiner des impuretés dans des récipients

(30) Priorität: 14.08.1991 DE 4126885
(43) Veröffentlichungstag der Anmeldung: 31.03.1993
(73) Patentinhaber: Rupp, Michael, D-75180 Pforzheim (DE)
(72) Erfinder: Rupp, Michael, D-75180 Pforzheim (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 306 307
- DE-A- 3 534 756
- DE-A- 4 038 993
- DE-U- 9 114 357
- US-A- 3 266 292
- US-A- 3 321 954
- US-A- 4 791 820

## Beschreibung

Die Erfindung betrifft ein Verfahren zum Untersuchen von Behältnissen auf Fremdstoffe nach dem Oberbegriff des Anspruchs 1 und eine Vorrichtung zur Untersuchung von Behältnissen auf Fremdstoffe nach dem Oberbegriff des Anspruchs 7.

Bei Mehrwegbehältnissen, wie insbesondere Mehrweggetränkeflaschen, vor allem aus Kunststoffen, wie PET-Material, stellt sich das Problem, daß sich in diesen Flaschen nicht Fremdstoffe, wie insbesondere fremde, schädliche Flüssigkeiten, befunden haben dürfen. Flaschen, in denen sich solche Fremdstoffe befunden haben, müssen ausgeschieden werden und dürfen nicht wiederverwendet werden.

Aus der EP-A-306 307 ist ein gattungsgemäßes Verfahren und eine gattungsgemäße Vorrichtung bekannt. Die Flaschen werden auf einen Rotationsförderer befördert und sind an ihrer Oberseite mit Ionisationskammern verbunden, in die Proben von in den Flaschen neu geformtem Dampf "gezogen" werden. Dies geschieht durch herkömmliche Pumpen, Venturi-Einrichtungen oder Gebläse mit oder ohne Vakuum-Akkumulatoren oder -zylinder. Die Probenentnahme erfolgt hier also durch Absaugen. Dies ist nachteilig und insbesondere nicht hinreichend wirksam. Auch beinhaltet die Ionisation eine Zerstörung der zu untersuchenden Fremdgase, so daß die Gefahr besteht, daß gewisse Moleküle als solche zerstört werden und damit nur die Bruchteile, nicht aber die Ausgangsgase festgestellt werden können.

Damit nur Proben von aus den Wandungen des Behältnisses neu herausdiffundierter Fremdstoffe entnommen werden, ist nach der Druckschrift weiterhin vorgesehen, daß zeitlich und räumlich vor der Probennahme zunächst in den Flaschen befindliches Gas durch Überdruck in die freie Umgebung ausgetrieben wird.

Die US-A-3,321,954 betrifft eine Vorrichtung zur Detektion von Verunreinigungen wie flüchtigen organischen Stoffen in Flaschen, Gläsern oder ähnlichen Behältern. Hierbei erfolgt die Probennahme mittels einer in die Flaschen einführbaren Entnahmeleitung durch "Abziehen" der Luftprobe aus den Flaschen. Hierzu wird eine herkömmliche Pumpe zum Ansaugen verwendet. Über die Entnahmeleitung gelangt die angesaugte Luft nun in eine gleichzeitig als Ionisations- und Detektionskammer dienende Meßküvette. Die Ionisation beinhaltet dabei eine Zerstörung der zu untersuchenden Fremdgase, so daß hierbei die Gefahr besteht, daß gewisse Moleküle als solche zerstört werden und damit nur die Bruchteile, nicht aber die Ausgangsgase festgestellt werden können. Des weiteren ist das Absaugen zur Probenentnahme nachteilig und insbesondere nicht hinreichend wirksam. Dies liegt daran, daß die Konzentration des Fremdstoffes beim Ansaugen nicht besonders hoch ist.

Ferner ist zur Überprüfung der Funktionsfähigkeit der Detektoreinrichtung ein Standard-Gas-System vorgesehen, mittels dessen eine Standardtestgasmischung von einer Gasquelle, die ein verunreinigtes Gas enthält, beispielsweise mit Cyclohexan verunreinigte Luft, über eine Leitung einer Haube zugeführt wird, in der sich die Entnahmeleitung in zurückgefahrener Stellung aus dem Behälter heraus befindet. Die Leitung zum Zuführen der Standardtestmischung ist dabei seitlich fest an der Haube angeordnet. Die sich dann innerhalb der Haube befindende Überprüfungsgasmischung wird dann über die Entnahmeleitung dem Detektorsystem zugeführt.

Der Erfindung liegt nun die Aufgabe zugrunde, ein Verfahren und eine Vorrichtung zu schaffen, die eine schnelle und äußerst zuverlässige Überprüfung von Behältern, insbesondere Flaschen, darauf ermöglichen, ob sich in ihnen störende Fremdstoffe befunden haben oder befinden.

Erfindungsgemäß wird die genannte Aufgabe bei einem Verfahren der eingangs genannten Art durch die kennzeichnenden Merkmale des Anspruchs 1 und bei einer Vorrichtung der oben genannten Art durch die kennzeichnenden Merkmale des Anspruchs 7 gelöst.

Bei dem erfindungsgemäßen Verfahren und der Vorrichtung ist also vorgesehen, daß das in dem Behältnis befindliche Gas durch die zentral durch die Lanze der Druckleitung eingeblasene Druckluft ausgetrieben wird, wobei das austretende Gas durch das zentrale Einblasen der Druckluft vorher seitlich an den Wandungen des Behältnisses entlangstreifen und von diesem Gaspartikel von Fremdstoffen aufnehmen und der Untersuchung zuführen kann. Durch das Arbeiten mit Überdruck, durch den die in der Flasche bzw. dem Behältnis befindliche Luft ausgetrieben wird, wird schneller eine höhere Konzentration des Fremdstoffes in die Meßküvette gebracht, als dies beim Absaugen wie beim Stand der Technik der Fall wäre. Damit sind schnellere Messungen und insgesamt ein höherer Durchsatz möglich. So hat man nämlich überraschend festgestellt, daß die Partikelkonzentration in der Meßküvette durch das Herausdrücken der in dem Behältnis wie einer Flasche befindlichen Luft gegenüber dem Absaugen deutlich erhöht werden kann. Durch den Überdruck werden die Gaspartikel einfach aus der Flasche heraus- und in die Meßküvette hineingetrieben. Durch diese Meßküvette werden dann die entnommenen Gasproben einer Detektoreinrichtung zugeführt. Es handelt sich also nicht bei der Meßküvette selbst um die Detektoreinrichtung. Die photometrische Untersuchung kann beispielsweise nach der DE-A-23 40 747 oder der WO 81/02633 erfolgen, die auch geeignete Detektoreinrichtungen zeigen.

In bevorzugter Weiterbildung kann vorgesehen sein, daß die Luft über eine in das Behältnis hineinbewegte Lanze eingeblasen und das austretende Gas über eine weitere Lanze der Meßküvette zugeführt wird, wobei insbesondere die Luft zentral in die Behältnisse eingeblasen wird. Eine weitere bevorzugte Ausgestaltung sieht vor, daß die Wandungen des Behältnisses zumindestens vor Einblasen der Luft erwärmt werden. Hierdurch wird die das in dem Behälter befindliche Gas austreibende Druckluft zentral eingeblasen, so daß das austretende Gas vorher an den Wandungen des Behälters entlangstreift und von diesem Gaspartikel von Fremdstoffen, die vorher gegebenenfalls durch die Erwärmung ausgetrieben wurden, aufnehmen und der Untersuchung zuführen kann.

Weiterbildungen der erfindungsgemäßen Vorrichtung sehen vor, daß eine Heizstation für die Behälter vorgesehen ist und daß die Heizstation IR-Strahler aufweist.

Gemäß einer weiteren Ausgestaltung der Erfindung ist vorgesehen, daß eine Küvette zur Aufnahme des aus einem Behälter herausgedrückten Gases vorgesehen und das aus dem Behälter herausgedrückte Gas in der Küvette in die Detetektoreinrichtung bringbar ist. Darüber hinaus zeichnet sich eine Weiterbildung dadurch aus, daß die Küvette mit Heizeinrichtungen versehen ist. Weitere Ausgestaltungen sehen vor, daß die Druckleitung eine Lanze aufweist, die zentral in einen Behälter einführbar ist und gekennzeichnet ist durch eine mit einer Ringdichtung versehenen Kappe, an dessen Umfang eine Entnahmeleitung für aus dem Behälter herausgedrücktes Gas mündet, die zu einer Meßküvette führt.

Gemäß einer Ausgestaltung ist vorgesehen, daß die Meßküvette stirnseitig mit dichten Sichtfenstern versehen ist. In diesem Falle weist die Meßküvette eine hohe Dichtigkeit auf, da sie allseitig abgedichtet ist, wobei der Einlaßzylinder und gegebenenfalls Auslässe durch Ventile vollständig abgedichtet werden können. Grundsätzlich und in diesem Falle ist weiterhin vorgesehen, daß die Küvette mit Spül-Auslässen versehen ist, in denen ein Ventil angeordnet ist.

In einer bevorzugten alternativen Ausgestaltung kann aber vorgesehen sein, daß die Meßküvette an beiden Stirnseiten offen ist und ihre Stirnseiten dicht an innen- und außenseitig angeordneten stationären Ringwandungen vorbeigeführt werden. Die Ringwandungen sind dabei bis auf einzelne Auslässe über den gesamten Umfang geschlossen. Diese Ausgestaltung weist den Vorteil auf, daß keine Sichtfenster vorhanden sind, die verschmutzen können und daher wiederholt gereinigt werden müssen, was zu unerwünschten Standzeiten führen kann. Bei dieser Ausgestaltung sieht eine bevorzugte Weiterbildung vor, daß Spülauslässe und gegebenenfalls Einlässe für die Meßküvette an geeigneten Stellen in den stationären Ringwandungen vorgesehen sind, nämlich dort, wo sich die Gasentnahmeeinrichtung zur Entnahme des Meßgases aus den zu untersuchenden Behältern befindet (im Bewegungsweg vor der Detektoreinrichtung) und dort, wo die Meßküvetten durch neutrales Spülgas von dem Meßgas wieder gereinigt werden (im Bewegungsweg unmittelbar hinter den Detektoreinrichtungen).

Die Spülluft kann dabei über den mit der Küvette umlaufenden Zuführeinlaß (durch den auch das Meßgas der Meßküvette zugeführt wird) zugeführt werden oder aber eben durch einen in einer Ringwandung stationär vorgesehenen Einlaß auf der einen Stirnseite der Meßküvette, während lediglich auf der gegenüberliegenden Stirnseite ein Auslaß angeordnet ist. Diese Ausgestaltungen weisen den Vorteil auf, daß separate Ventile entfallen können, da die Stirnseiten automatisch hinreichend verschlossen werden, wenn sie aus den Ein- und Auslaßbereichen in den Wandungen heraus zu den geschlossenen Bereichen der umgebenden Wandungen bewegt werden. Der Spalt zwischen den offenen Stirnseiten der Meßküvette und den umgebenden geschlossenen Ringwandungen weist lediglich eine Stärke von wenigen Hundertstel Millimetern auf. Es hat sich herausgestellt, daß der Verlust von Meßgas vernachlässigbar ist.

Durch die Erfindung wird erreicht, daß Behälter, wie PET-Flaschen, Gasflaschen oder auch andere Behältnisse, auf in ihnen befindliche gasförmige Fremdstoffe, wie Haarwasser, Öle oder dergleichen, aber auch Verwesungsgase von in die Flaschen eingebrachten Kleintieren oder dergleichen zuverlässig und schnell festgestellt werden können. Darüber hinaus kann die Erfindung auch zur Qualitätssicherung im Produktionsprozeß eingesetzt werden. Durch das Arbeiten mit Überdruck, durch den die in der Flasche befindliche Luft ausgetrieben wird, wird schneller eine höhere Konzentration des Fremdstoffes in die Meßküvette gebracht, als dies beim Absaugen der Fall wäre. Es sind damit schnellere Messungen und insgesamt ein höherer Durchsatz möglich. So hat man festgestellt, daß die Partikelkonzentration in der Meßküvette durch das Herausdrücken der in der Flasche befindlichen Luft gegenüber dem Absaugen deutlich erhöht werden kann. Dies wird dadurch unterstützt, daß die Behälterwandung vor dem Herausdrücken erwärmt wird. Es hat sich gezeigt, daß IR-Licht, beispielsweise in PET-Flaschen, zu 99 % ab sorbiert wird, damit die Flaschenwandung erwärmt. Durch die Erwärmung werden aufgrund des bedingten höheren Dampfdrucks in die Wandung hineindiffundierte Gasmoleküle wieder aus diesen heraus in das Innere des Behältnisses gebracht. Durch den Überdruck werden sie aus der Flasche aus- und in die Meßküvette eingetrieben.

Weitere Vorteile und Merkmale der Erfindung ergeben sich aus den Ansprüchen und aus der nachfolgenden Beschreibung, der ein Ausführungsbeispiel der erfindungsgemäßen Vorrichtung zum Untersuchen von Behältnissen auf Fremdstoffe unter Bezugnahme auf die Zeichnung im einzelnen erläutert ist. Dabei zeigt:
- Figur 1: eine schematische Gesamtdarstellung der bevorzugten Ausführungsform der erfindungsgemäßen Vorrichtung in Draufsicht;
- Figur 2: eine Seitenansicht, teilweise geschnitten der wesentlichen erfinderischen Teile der erfindungsgemäßen Vorrichtung;
- Figur 3: einen Schnitt durch eine weitere Ausgestaltung der erfindungsgemäßen Vorrichtung mit stirnseitig offener Meßküvette im Bereich von stationären Gasauslässen; und
- Figur 4: einen Schnitt durch die Ausgestaltung der Figur 3 im Bereich einer Detektoreinrichtung.

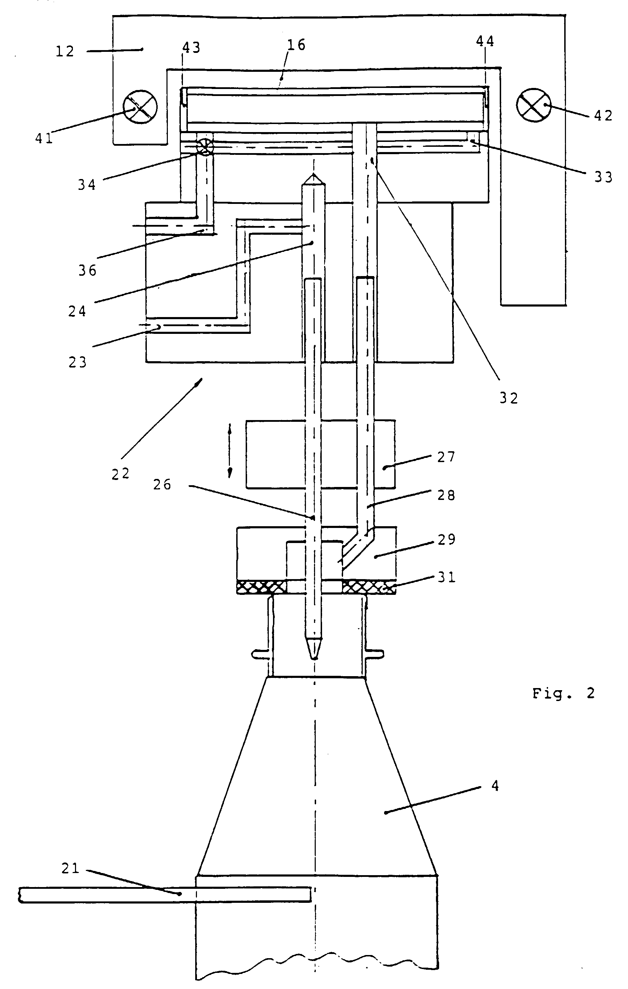

Die erfindungsgemäße Vorrichtung 1 weist ein Grundgestell 2 auf. Auf dem Grundgestell befindet sich eine Zuführbahn 3 für die zu untersuchenden Behältnisse 4 (Figur 2), wie Flaschen, insbesondere PET-Flaschen. Weiterhin ist eine Fortführbahn 6 vorgesehen, die bei der dargestellten Ausführungsform der erfindungsgemäßen Vorrichtung parallel zur Zuführbahn verläuft, die Behältnisse 4 aber in Gegenrichtung aus der erfindungsgemäßen Vorrichtung herausfördert. In beiden Bahnen 3, 6 ist jeweils ein ÜbergabeKreisförderer 7, 8 zugeordnet. Zwischen den beiden Kreisförderern 7, 8 befindet sich die eigentliche Untersuchungseinrichtung 9, die ebenfalls einen Kreisförderer 11 aufweist und an deren Umfang eine Detektoreinrichtung 12 angeordnet ist. Beidseits der Zuführbahn 3 befindet sich eine Heizeinrichtung 13, beispielsweise in Form von Infrarotstrahlern, die sich über die Höhe der Behältnisse 4 erstrecken.

Im Umfang des Kreisförderers 11 sind Aufnahmen 14 für die Behältnisse 4 ausgebildet. Oberhalb der Aufnahmen 14 befinden sich, jeder Aufnahme 14 zugeordnet, jeweils eine Küvette 16, die durch die Detektoreinrichtungen 12 laufen, so daß dort das in den Küvetten 16 befindliche Gas, welches in noch zu beschreibender Weise aus den Behältnissen 4 entnommen wird, auf Fremdbestandteile untersucht werden kann. Den Küvetten 16 können weiter am Kreisförderer 11 ebenfalls Heizeinrichtungen zugeordnet sein (nicht dargestellt), um in den Küvetten befindliches Gas auszutreiben und diese für eine weitere Probennahme aus einem anderen Behältnis vorzubereiten.

Die Behältnisse werden über die Zuführbahn 3 zugeführt. Ihre Wandungen werden durch die Heizeinrichtung 13 erwärmt, so daß in die Wandungen diffundierte Fremdgasanteile, insbesondere von Fremdstoffen, wie Festkörpern, Flüssigkeiten oder auch Gasen, die sich früher in den Flaschen befunden haben, wieder in das Flascheninnere austreten. Die Flaschen werden vom Übergabe-Kreisförderer 7 übernommen und dem Kreisförderer 11 übergeben. Dort wird in der weiter unten zu beschreibenden Weise eine Gasprobe genommen, in die der Flasche zugeordnete Küvette 16 geleitet und, sobald diese durch die Detektoreinrichtung 12 tritt, in dieser analysiert. Anschließend werden die Flaschen vom Förderer 11 auf den Übergabe-Kreisförderer 8 und von diesem der Fortführbahn 6 übergeben. Der Übergabe-Kreisförderer 8 ist mit einer durch die Detektoreinrichtung 12 gesteuerten Steuereinrichtung versehen, mittels derer nur unbedenkliche Behältnisse auf die Fortführbahn 6 übergeben werden, während als bedenklich festgestellte Behältnisse in einer Flaschenabwurfstation 17 ausgeworfen werden.

Die Behältnisse 4 werden durch Halterungen 21, wie Greifer oder dergleichen, am Umfang des Kreisförderers 11 in den Aufnahmen 14 gehalten. Oberhalb der Flaschen bzw. der Aufnahmen 14 befindet sich eine Gasentnahmeeinrichtung 22. Diese weist eine in einen Zylinder 24 führende Druckluftleitung 23 auf. Der Zylinder 24 befindet sich koaxial über dem durch die Halter 21 gehaltenen Behältnis 4. In den Zylinder 24 ragt, dicht einsitzend, im Öffnungsbereich des Behälters 24 eine Druckluftlanze 26, die mit ihrem unteren Ende in das Behältnis 4 einführbar ist. Sie wird durch einen verfahrbaren Block 27 gehalten, der am Umfang des Kreisförderer 11 auf- und abfahrbar ist, wobei das obere Ende der Lanze 26 mehr oder weniger tief in den Zylinder 24 eingefahren wird und dabei aus den Behältnissen 4 heraus- bzw. in diese hereingefahren wird. Mit dem Block 27 ist weiterhin eine Gasentnahmeleitung in Form einer Gasentnahmelanze 28 verbunden, die an ihrem unteren Ende in eine Kappe 29 mündet, welche an ihrer unteren Stirnseite mit einer Ringdichtung 31 versehen ist, die bei Abwärtsbewegung des Blocks 27 fest und dichtend auf dem oberen Rand des Behältnisses 4 aufsetzbar ist. Die Lanze 28 ragt ebenfalls in einen Zylinder 32, der von seiner oberen Seite in die Küvette 16 mündet. Die Küvette 16 ist mit zwei Auslässen 33 versehen, die über ein Ventil 34 mit einer Auslaßleitung 36 in Verbindung bringbar sind. Die Druckluftleitung 23 ist an eine (nicht dargestellte) Druckluftquelle anschaltbar.

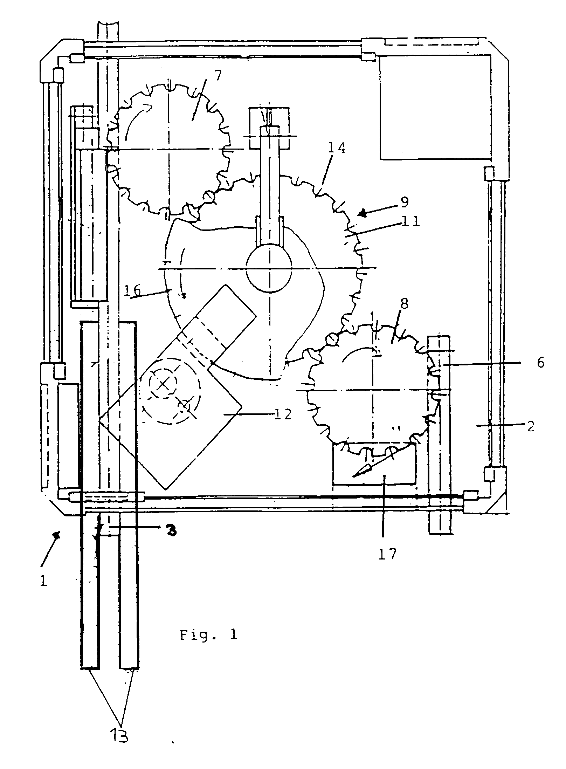

Wenn die in der Heizstation 13 erwärmten Behältnisse 4 vom Übergabe-Kreisförderer 7 zum Kreisförderer 11 übernommen wurden (Figur 1), verfährt der Block 27, der die Lanzen 26, 28 trägt, aus einer oberen Stellung nach unten und führt dabei das untere Ende der Lanze 26 in die Öffnungen des Behältnisses 4 ein, bis die Dichtung' 31 der Kappe 29 fest auf dem Rand der Öffnung des Behältnisses 4 aufliegt. Anschließend wird von der Druckgasquelle (nicht dargestellt) über die Druckluftleitung 23, den Zylinder 24 und die Lanze 26 unter Druck stehendes Gas, das gegebenenfalls auch Druckluft sein kann, in die Flasche 24 gedrückt. Durch die über die Lanze 26 in das Behältnis 4 eingedrückte Druckluft oder das eingedrückte Druckgas wird die in der Flasche befindliche Luft mit den gasförmigen Verunreinigungsanteilen, die vorher durch die Heizeinrichtung 13 aus der Wandung der Flasche in das Innere getrieben wurden, aus dem Behältnis über die Lanze 28 aus- und in die Küvette 16 getrieben. Dabei wird in der Küvette befindliches neutrales Spülgas und gegebenenfalls auch zunächst der erste Anteil des an der Flasche getrichterten Meßgases aus der Küvette 16 durch die Auslässe 33 verdrängt, bis das Ventil 34 geschlossen wird.

Gelangt die Küvette 16 nun in die Detektoreinrichtung 12, so wird in der Küvette 16 befindliches Gas durch die Detektoreinrichtung 12 untersucht. Die Detektoreinrichtung 12 weist gegebenenfalls mehrere Lichtquellen 41 und Detektoren 42 auf. Die Küvette 16 ist hierzu an ihren Stirnseiten mit Sichtfenstern 43, 44 versehen. Gegebenenfalls kann die Detektoreinrichtung 12 mehrere Einheiten aufweisen, die für verschiedene Gasanteile und deren Absorptionsbanden vorgesehen sind. Wenn durch Weiterbewegung des Kreisförderers 11 die Küvette aus der Detektoreinrichtung herausgebracht wurde, so kann sie durch Luft oder ein neutrales Gas - über den Einlaßzylinder 32 und die Auslässe 33 - gespült werden, wobei sie ebenfalls zur Unterstützung des Austreibens des aus dem Behältnis entnommenen Gases erwärmt werden kann. Die Kreisförderer arbeiten intermittierend. Im weiteren Bewegungsablauf wird der Block 27 angehoben, löst damit die Dichtung 31 von der Öffnung des Behältnisses 4 und zieht das untere Ende der Lanze 26 aus dem Behältnis 4, so daß dieses dann an den Übergabe-Kreisförderer 9 übergeben werden kann. Es wird von diesem dann entweder auf die Fortführbahn übergeben oder aber durch die Abwurfeinrichtung eliminiert.

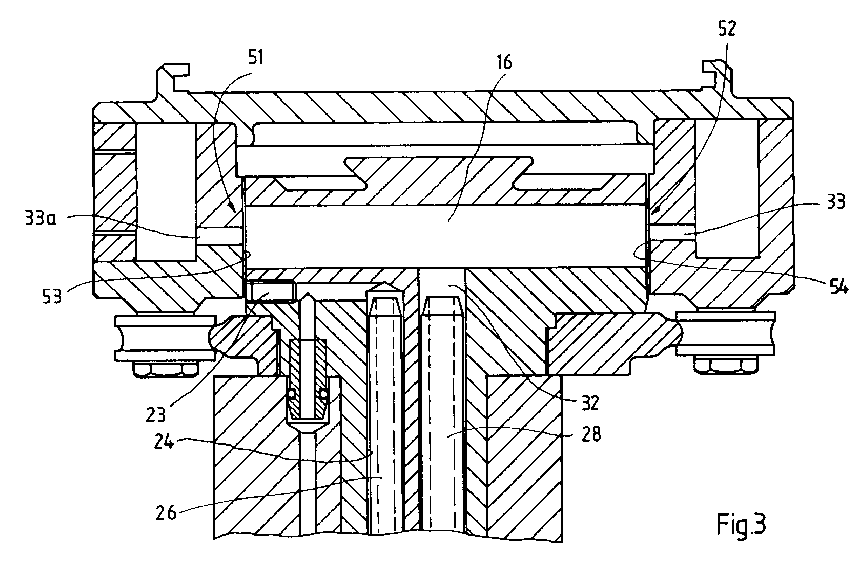

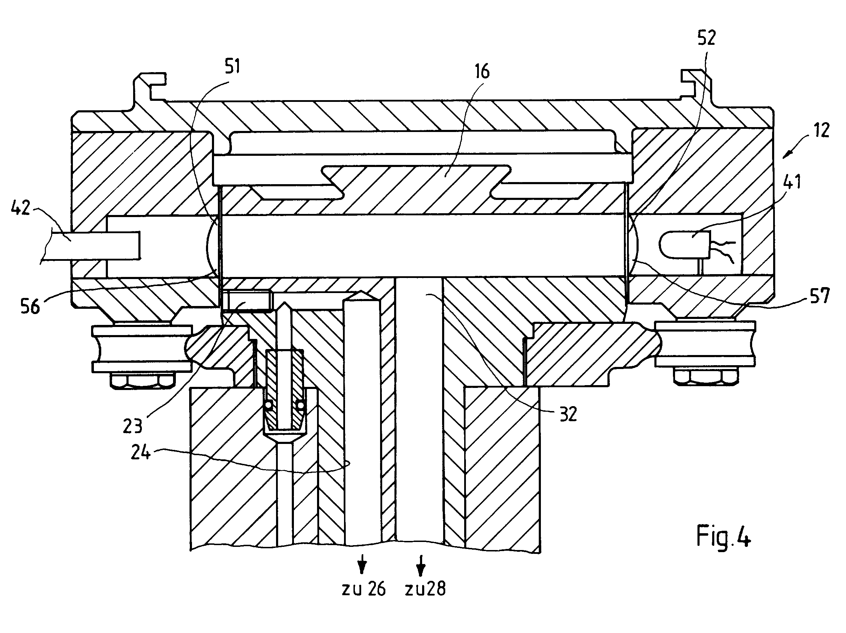

Die Figuren 3 und 4 zeigen Schnitte einer weiteren bevorzugten Ausgestaltung der erfindungsgemäßen Vorrichtung, wobei die Figur 3 einen Schnitt im Bereich von Spülauslässen 33 und die Figur 4 einen Schnitt im Bereich einer Detektoreinrichtung 12 zeigt.

Bei dieser Ausgestaltung sind die Stirnseiten 51, 52 der Meßküvette 16 nicht mit Sichtfenstern versehen, sondern vielmehr offen ausgebildet. Die Stirnseiten 51, 52 der Meßküvette 16 werden dabei zumindestens über den Umfang des Kreisförderers in dem Bereich, in dem die Küvette Meßgas enthält, vorzugsweise aber auch in dem Bereich, der der Spülstation mit Neutralgas folgt, durch weitgehend geschlossene innere und äußere Wandungen 53, 54 abgedeckt. Der Spalt zwischen den Stirnseiten 51, 52 der Küvette 16 und den stationären Wandungen 53, 54 liegt dabei im Bereich von wenigen Hundertstel Millimeter, beispielsweise zwischen 20 und 50 Mikrometern. Es hat sich schon herausgestellt, daß der Verlust von in der Küvette 16 befindlichem Meßgas durch diese Spalte vernachlässigbar ist. Bei der Ausgestaltung der Figur 3 befindet sich die Küvette 16 im Bereich der Spülstation für die Küvette 16 mit Öffnungen 33, 33a in den sie innen und außen umgebenden Wandungen 53, 54. Die Öffnung 33 ist dabei ein Spülgasauslaß 33, während die Öffnung 33a entweder ebenfalls ein solcher Auslaß oder ein Spüllufteinlaß sein kann. Im ersteren Falle (wenn 33a auch ein Auslaß ist), so kann die Spülluft über die mit der Küvette umlaufende Probeentnahmelanze 28 und den Zuführzylinder 32 zugeführt werden, wobei die Spülluft diesen dann in geeigneter Weise (über eine stationäre Langlochöffnung) zugeführt wird.

Die Ausgestaltung der Vorrichtung im Bereich der Probeentnahmestation ist im wesentlichen die gleiche, wobei dort allerdings die mit der Küvette 16 umlaufende Druckluftleitung 23 an einem entsprechenden stationären Zuführeinlaßbereich vorbeigeführt wird.

Die Figur 4 zeigt einen Schnitt im Bereich der Detektoreinrichtung. Die Detektoreinrichtung weist ebenfalls wie bei der Ausgestaltung der Figur 2 eine Lichtquelle 41 und einen Detektor 42 auf (gegebenenfalls können mehrere Detektoreinrichtungen 12 für verschiedene Fremdgasanteile hintereinander angeordnet sein). Es ist weiterhin eine Optik mit Linsen 56, 57 vorgesehen, durch die das von der Lichtquelle 41 ausgehende Licht durch die Küvette 16 hindurch parallelisiert bzw. auf den Detektor 42 fokussiert wird.

Soweit einzelne Elemente bei der Erläuterung der Figuren 3 und 4 nicht besonders erwähnt wurden, so sind sie ebenso wie bei der Ausgestaltung der Figur 2 ausgebildet.

## Patentansprüche

1. Verfahren zum Untersuchen von Behältnissen auf Fremdstoffe, wobei aus dem Behältnis eine Gasprobe entnommen und untersucht wird, dadurch gekennzeichnet daß die Gasprobe derart entnommen wird, daß in das Behältnis Luft eingeblasen wird, um in dem Behältnis befindliches Gas in eine Meßküvette auszutreiben und derart das vorher in dem Behältnis befindliche und aus diesem durch den bewirkten Überdruck austretende Gas in der Meßküvette photometrisch untersucht wird.

2. Verfahren nach Anspruch 1, dadurch gekennzeichnet, daß die Wandungen des Behältnisses zumindestens vor Einblasen der Luft erwärmt werden.

3. Verfahren nach Anspruch 1 oder 2, dadurch gekennzeichnet, daß die Luft über eine in das Behältnis hineinbewegte Lanze eingeblasen und das austretende Gas über eine weitere Lanze der Meßküvette zugeführt wird.

4. Verfahren nach einem der vorangehenden Ansprüche, dadurch gekennzeichnet, daß die Luft zentral in die Behältnisse eingeblasen wird.

5. Verfahren nach einem der vorangehenden Ansprüche, dadurch gekennzeichnet, daß die Meßküvette zwischen Messungen mit einem neutralen Spülgas, wie der Luft, gespült werden.

6. Verfahren nach Anspruch 5, dadurch gekennzeichnet, daß eine Meßküvette beim Spülen erwärmt wird.

7. Vorrichtung zur Untersuchung von Behältnissen auf Fremdstoffe mit einer von einer Öffnung des Behältnisses zu einer Meßküvette führenden Entnahmeleitung und einer von einer Gasquelle in das Behältnis führenden Druckleitung, dadurch gekennzeichnet, daß die Gasquelle eine Druckluftquelle ist, die Druckleitung (23) eine zentral in das Behältnis (4) einführbare Lanze (26) aufweist und die Meßküvette (16) zur Aufnahme der aus dem Behältnis (4) herausgedrückten Gasprobe vorgesehen und die aus dem Behältnis (4) herausgedrückte Gasprobe in der Meßküvette (16) in eine Detektoreinrichtung (12) bringbar ist.

8. Vorrichtung nach Anspruch 7, dadurch gekennzeichnet, daß eine Heizstation (13) für die Behälter (4) vorgesehen ist.

9. Vorrichtung nach Anspruch 8, dadurch gekennzeichnet, daß die Heizstation (13) IR-Strahler aufweist.

10. Vorrichtung nach einem der Ansprüche 7 bis 9, dadurch gekennzeichnet, daß die Küvette (16) mit Heizeinrichtungen versehen ist.

11. Vorrichtung nach einem der Ansprüche 7 bis 10, gekennzeichnet durch eine mit einer Ringdichtung (31) versehenen Kappe (29), an dessen Umfang eine Entnahmeleitung für aus dem Behälter (4) herausgedrücktes Gas mündet, die zu einer Meßküvette (16) führt.

12. Vorrichtung nach einem der Ansprüche 7 bis 11, dadurch gekennzeichnet, daß die Meßküvette (16) stirnseitig mit dichten Sichtfenstern (43, 44) versehen ist.

13. Vorrichtung nach einem der Ansprüche 7 bis 12, dadurch gekennzeichnet, daß die Küvette (16) mit Spülauslässen (33) versehen ist, in denen ein Ventil (34) angeordnet ist.

14. Vorrichtung nach einem der Ansprüche 7 bis 11, dadurch gekennzeichnet, daß die Meßküvette (16) stirnseitig offen ist und ihre Stirnseiten dicht an innen- und außenseitig angeordneten, stationären Ringwandungen vorbeigeführt werden.

15. Vorrichtung nach Anspruch 14, dadurch gekennzeichnet, daß Spülauslässe (33) und gegebenenfalls Einlässe für die Küvette (16) in den stationären Ringwandungen vorgesehen sind.

## Claims

1. Method for examining containers for impurities, a gas sample being removed from the container and examined, characterized in that the gas sample is removed in such a way that air is blown into the container so as to expel the gas in the container into a measuring cell and so that gas previously in the container and discharged therefrom through the overpressure obtained is photometrically examined in the measuring cell.

2. Method according to claim 1, characterized in that the walls of the container are heated at least prior to the blowing in of air.

3. Method according to claim 1 or 2, characterized in that air is blown in by means of a lance moved into the container and the discharged gas is supplied via a further lance to the measuring cell.

4. Method according to one of the preceding claims, characterized in that air is blown centrally into the containers.

5. Method according to one of the preceding claims, characterized in that between measurements the measuring cell is scavenged with a neutral scavenging gas, such as air.

6. Method according to claim 5, characterized in that a measuring cell is heated during scavenging.

7. Device for examining containers for impurities with a discharge line leading from a container opening to a measuring cell and a pressure line leading from a gas source into the container, characterized in that the gas source is a compressed air source, the pressure line (23) has a lance (26) centrally insertable into the container (4) and the measuring cell (16) is provided for receiving the gas sample forced out of the container (4) and the gas sample forced out of the container (4) can be brought into a detector means (12) in the measuring cell (16).

8. Device according to claim 7, characterized in that a heating station (13) is provided for the container (4).

9. Device according to claim 8, characterized in that the heating station (13) has a IR lamp.

10. Device according to one of the claims 7 to 9, characterized in that the cell (16) is provided with heating devices.

11. Device according to one of the claims 7 to 10, characterized by a cap (29) provided with a ring seal (31) and at whose circumference issues a discharge line for gas forced out of the container (4) and leading to a measuring cell (16).

12. Device according to one of the claims 7 to 11, characterized in that the measuring cell (16) is frontally provided with tight viewing windows (43, 44).

13. Device according to one of the claims 7 to 12, characterized in that the cell (16) is provided with scavenging outlets (33) in which a valve (34) is located.

14. Device according to one of the claims 7 to 11, characterized in that the measuring cell (16) is frontally open and its front faces are tightly moved past internally and externally positioned, stationary annular walls.

15. Device according to claim 14, characterized in that scavenging outlets (33) and optionally inlets for the cell (16) are provided in the stationary annular walls.

## Revendications

1. Procédé d'examen de récipients en vue d'y détecter des impuretés, selon lequel on prélève dans le récipient un échantillon de gaz et on l'examine, caractérisé en ce que le prélèvement de l'échantillon est réalisé par une injection d'air dans le récipient qui expulse le gaz contenu dans le récipient dans une chambre de mesure et pour que le gaz préalablement contenu dans le récipient et expulsé de celui-ci par la pression exercée soit analysé par photométrie.

2. Procédé selon la revendication 1, caractérisé en ce que les parois du récipient sont réchauffées au moins avant l'injection de l'air.

3. Procédé selon l'une quelconque des revendications 1 ou 2, caractérisé en ce que l'air est injecté par une lance introduite dans le récipient et en ce que le gaz expulsé est amené dans la chambre de mesure par une autre lance.

4. Procédé selon l'une quelconque des revendications précédentes, caractérisé en ce que l'air est injecté dans les récipients de manière centrale.

5. Procédé selon l'une quelconque des revendications précédentes, caractérisé en ce que entre les mesures, on lave la chambre de mesure avec un gaz inerte de lavage, tel que de l'air.

6. Procédé selon la revendication 5, caractérisé en ce que l'on chauffe la chambre lors du lavage.

7. Dispositif pour examiner des récipients en vue d'y détecter des impuretés, comprenant une conduit de prélèvement s'étendant d'une ouverture du récipient vers une chambre de mesure et une conduite forcée reliant une source de gaz au récipient, caractérisé en ce que la source de gaz est une source d'air comprimé, en ce que la conduite forcée (23) comporte une lance (26) introduite de manière centrale dans le récipient (4), en ce que la chambre de mesure (16) est prévue pour la réception de l'échantillon de gaz expulsé du récipient (4) et en ce que l'échantillon de gaz expulsé du récipient (4) est introduit dans un dispositif de détection (12) situé dans la chambre de mesure (16).

8. Dispositif selon la revendication 7, caractérisé en ce que l'on prévoit un dispositif de chauffage (13) pour les récipients (4).

9. Dispositif selon la revendication 8, caractérisé en ce que le dispositif de chauffage (13) présente un élément à rayonnement infrarouge.

10. Dispositif selon l'une quelconque des revendications 7 à 9, caractérisé en ce que la chambre de mesure (16) est pourvue de dispositifs de chauffage.

11. Dispositif selon l'une quelconque des revendications 7 à 10, caractérisé par une calotte (29) à joint annulaire (31), à la périphérie de laquelle débouche une conduite de prélèvement de gaz expulsé du récipient (4) vers une chambre de mesure (16).

12. Dispositif selon l'une quelconque des revendications 7 à 11, caractérisé en ce que la chambre de mesure (16) est pourvue sur sa face frontale de fenêtres d'observation (43,44) étanches.

13. Dispositif selon l'une quelconque des revendications 7 à 12, caractérisé en ce que la chambre (16) comporte des vidages de nettoyage (33) dans lesquelles est disposée une soupape (34).

14. Dispositif selon l'une quelconque des revendications précédentes, selon l'une quelconque des revendications 7 à 11, caractérisé en ce que la chambre de mesure (16) est ouverte frontalement et en ce que ses faces frontales sont déplacées à guidage de façon étanche devant les parois annulaires disposées fixes à l'intérieur et à l'extérieur.

15. Dispositif selon la revendication 14, caractérisé en ce que les vidages de nettoyage (33) et le cas échéant les entrées pour la chambre (16) sont prévues dans les parois annulaires fixes.
